# EUROPÄISCHE PATENTSCHRIFT

(11) **EP 0 645 389 B1**
(45) Veröffentlichungstag und Bekanntmachung des Hinweises auf die Patenterteilung: **05.02.1997**
(21) Anmeldenummer: 94114551.8
(22) Anmeldetag: 15.09.1994
(51) Int. Cl.: C07D 471/04

(54) **Verfahren zur Herstellung von Imidazopyridinderivaten**
Process for the production of imidazopyridines
Procédé pour la préparation de derivés des imidazopyridines

(30) Priorität: 17.09.1993 CH 2816/93
(43) Veröffentlichungstag der Anmeldung: 29.03.1995
(73) Patentinhaber: LONZA AG, CH-3945 Gampel/Wallis (CH)
(72) Erfinder: Stucky, Gerhard, Dr., CH-3902 Brig-Glis (Kanton Wallis) (CH); Imwinkelried, René, Dr., CH-3902 Brig-Glis (Kanton Wallis) (CH)
(74) Vertreter: Weinhold, Peter, Dr.

(56) Entgegenhaltungen:
- EP-A- 0 130 461
- EP-A- 0 385 850
- EP-A- 0 510 813
- WO-A-93/23399
- US-A- 5 066 654
- US-A- 5 240 938

## Beschreibung

Die Erfindung betrifft ein neues Verfahren zur Herstellung von Imidazopyridinderivaten der allgemeinen Formel 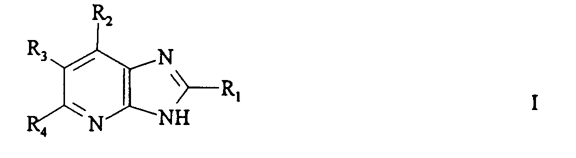
worin
- R₁: eine Alkyl-, Cycloalkyl-, Aryl- oder Aralkylgruppe bedeutet oder für einen heterocyclischen Rest steht,
- R₂ und R₄: gleich oder verschieden sind und Wasserstoff, eine Hydroxy-, eine Cyan-, Alkyl-, Cycloalkyl-, Aryl- oder Aralkylgruppe bedeuten oder für eine Alkanoyl - oder eine Alkoxycarbonylgruppe stehen und
- R₃: Wasserstoff, eine Alkyl-, Aryl- oder Aralkylgruppe oder ein Halogenatom bedeutet.

Diese Verbindungen finden Anwendung als Zwischenprodukte für die Herstellung von Angiotensin-II-Antagonisten (J. Med. Chem 1991, 34, 2919 - 2922).

In der genannten Literaturstelle wird beschrieben, dass die Imidazopyridine durch Reduktion von 2-Amino-3-nitropyridinen und durch anschliessende Kondensation mit einer entsprechenden aliphatischen Carbonsäure erhalten werden können.

Die Bereitstellung der Ausgangsprodukte der 2-Amino-3-nitropyridine gestaltet sich hingegen schwierig, da die Nitrierung der entsprechenden Aminopyridine nicht regioselektiv verläuft.

Die Aufgabe der Erfindung bestand folglich darin ein Verfahren zu entwickeln, dass einen einfachen und grosstechnisch umsetzbaren Zugang zu den Imidazopyridinen ermöglicht.

Die Aufgabe konnte gelöst werden mit dem Verfahren gemäss Anspruch 1.

Die für die einzelnen Reste R₁ bis R₇ verwendeten Begriffe haben nachfolgende Bedeutung.

Unter der Bezeichnung Alkylgruppe wird eine geradkettige oder verzweigte Alkylgruppe mit zweckmässig 1 bis 6 C-Atomen, vorzugsweise mit 1 bis 4 C-Atomen, verstanden.

Beispielhaft seien die Methyl-, Ethyl-, n-Propyl-, i-Propyl-, n-Butyl- der die t-Butylgruppe genannt.

Unter der Bezeichnung Cycloalkylgruppe wird zweckmässig eine C₃-C₆-Cycloalkylgruppe, wie z. B. eine Cyclopropyl, eine Cyclobutyl-, eine Cyclopentyl- oder eine Cyclohexylgruppe, verstanden.

Die Bezeichnung Aryl umfasst carbocyclische Aromaten, zweckmässig Phenyl oder Naphthyl, und die Bezeichnung Aralkyl steht für eine arylsubstituierte Alkylgruppe, zweckmässig für eine phenylsubstituierte C₁-C₆-Alkylgruppe,insbesondere für Benzyl.

Unter einer Alkanoylgruppe wird zweckmässig eine (C₁-C₆)-Alkanoylgruppe,vorzugsweise Acetyl,verstanden.

Alkoxy bedeutet zweckmässig (C₁-C₆)-Alkoxy,vorzugsweise Methoxy oder Ethoxy.

Unter einem heterocyclischen Rest wird zweckmässig ein 5- oder 6-Ringheterocyclus mit Stickstoff und / oder Sauerstoff und / oder Schwefel als Heteroatom verstanden. Ebenso zählen kondensierte Ringsysteme von Heterocyclen untereinander oder von Heterocyclen mit carbocyclischen. Systemen unter den genannten Begriff. Beispielhaft für 5-Ring-Heterocyclen seien die Furane, die Thiophene, die Pyrrole, die Indole, die Pyrazole, die Imidazole, die Oxazole, Isoxazole, die Thiazole oder die Triazole genannt.

Als Beispiel für 6-Ring-Heterocyclen werden die Pyridine, die Chinoline, die Isochinoline, die Acridine, die Pyridazine, die Pyrimidine, die Pyrazine, die Phenazine, die Purine oder die Pteridine genannt.

Unter Halogen wird Fluor, Chlor, Brom oder Jod verstanden, bevorzugtes Halogen ist Chlor.

Die genannten Gruppen, insbesondere die cyclischen Reste, können jeweils einfach oder mehrfach substituiert sein. Geeignete Reste sind z. B. Halogen, Nitro, Amino, Alkylamino, Dialkylamino, Hydroxy, Alkoxy, Alkyl oder Alkanoyl. Für die Bedeutung dieser Reste sind die vorstehenden Erklärungen gültig.

Die Herstellung des Ausgangsprodukts des erfindungsgemässen Verfahrens, des Alkoxyimidats der allgemeinen Formel 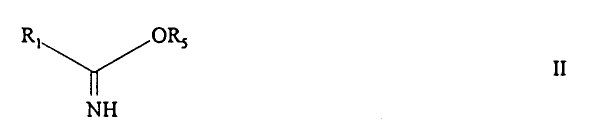
worin R₁ die genannte Bedeutung hat und R₅ Alkyl, Aryl oder Aralkyl bedeutet, kann analog Brooker et al. J. Am. Chem. Soc. 1935, 57, 2480ff durch Umsetzung eines Nitrils der allgemeinen Formel

R₁CN IV

mit einem Alkohol der allgemeinen Formel

R₅OH V

worin R₅ die genannte Bedeutung hat,in Gegenwart von einem Halogenwasserstoff zum Alkoxyimidathydrohalogenid und anschliessende Freisetzung des Alkoxyimidats mit einer Base erfolgen.

Zweckmässig wird als Nitril der allgemeinen Formel IV Acetonitril, Propionitril, Butyronitril oder Valeronitril,bevorzugt Propionitril,eingesetzt.

Geeignete aliphatische Alkohole der allgemeinen Formel V sind Methanol, Ethanol, n- oder i-Propanol, n-, i- oder t-Butanol,bevorzugt Methanol.

Bevorzugter Halogenwasserstoff ist Chlorwasserstoff.

Als Lösungsmittel kann der eingesetzte Alkohol R₅OH fungieren. Es ist aber auch möglich, ein zusätzliches inertes Lösungsmittel, wie z. B. einen Ether, wie Dioxan oder Diethylether, oder einen aromatischen Kohlenwasserstoff, wie Toluol,einzusetzten.

Das resultierende Alkoxyimidat der allgemeinen Formel II kann auf fachmännische Weise aus dem Reaktionsgemisch isoliert werden, von Vorteil wird es gelöst, in einem der genannten Lösungsmittel, der Folgestufe zugeführt.

Erfindungsgemäss wird in der Folgestufe das Alkoxyimidat der allgemeinen Formel II mit Aminoacetonitril und mit einer 1,3-Dicarbonylverbindung der allgemeinen Formel 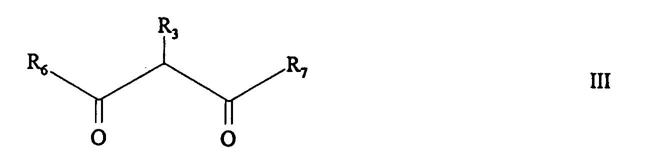
worin R₃ die genannte Bedeutung hat und R₆ und R₇ gleich oder verschieden sind und Wasserstoff oder Alkyl, Aryl, Aralkyl, Alkoxy oder Alkoxycarbonyl bedeuten, zum Endprodukt ringgeschlossen.

Geeignete 1,3-Dicarbonylverbindungen mit R₆ und R₇ = Alkyl sind die Alkandione wie z. B. das 2,4-Pentandion (Acetylaceton), das 3,5-Heptandion, das 4,6-Nonandion oder das 3-Methyl-2,4-pentandion (2-Methylacetylaceton) mit R₃ = Methyl.

Vertreter mit R₆ = Alkyl und R₇ = Alkoxy sind die Alkanoylessigsäureester wie z. B. der Acetessigsäuremethylester oder der Acetessigsäureethylester.

Zweckmässig werden auch die Malonester mit R₆ und R₇ = Alkoxy eingesetzt. Beispielhaft seien der Malonsäuremethylester und der Malonsäureethylester genannt.

Geeignete Verbindungen der allgemeinen Formel III mit R₆ und R₇ = Wasserstoff sind das Malondialdehyd bzw. die 2-substituierten Malondialdehyde.

Weitere geeignete Vertreter der allgemeinen Formel III mit R₆ und R₇ = Alkoxycarbonyl sind der 2,4-Dioxopentandisäuredimethylester oder der 2,4-Dioxopentandisäurediethylester mit R₆ und R₇ = Methoxycarbonyl bzw. Ethoxycarbonyl.

Das Aminoacetonitril kann jeweils direkt vor der Reaktion aus einem entsprechenden Aminoacetonitrilsalz, wie z. B. dem Hydrochiorid oder dem Hydrosulfat, durch dessen Umsetzung mit einer Base, z. B. mit Animoniak, freigesetzt werden.

Es ist aber auch möglich,das Aminoacetonitrilsalz im Verlaufe der Reaktion in Form z. B. einer Suspension zusammen mit einer Base zuzusetzen.
Als Base können z .B. ein Aikaliaikoholat wie z B. Na- oder K-ethanolat, Na-/K-methanolat oder K-t-butylat im entsprechenden Alkohol, ein Trialkylamin wie z. B. Triethylamin oder Ethyldiisopropylamin, ein Alkalihydroxid wie z. B. NaOH oder KOH in einem aliphatischen Alkohol oder Wasser, aber auch Alkali- oder Erdalkalihydrogencarbonate in Wasser verwendet werden.

Gegebenenfalls kann die 1,3-Dicarbonylverbindung als Lösungsmittel fungieren, so dass ein zusätzliches Lösungsmittel prinzipiell nicht notwendig wird. Eine allfällige Lösungsmittelwahl ist nicht sonderlich kritisch. Gute Resultate können mit niederen aliphatischen Alkoholen wie z. B. Methanol oder Ethanol, halogenierten Kohlenwasserstoffen wie z. B. Methylenchlorid, Ether wie z. B. Dioxan oder mit aromatischen Kohlenwasserstoffen wie z. B. Toluol oder Xylol erhalten werden.

Die Umsetzung verläuft zweckmässig zwischen Raumtemperatur und Rücktlusstemperatur des jeweiligen Lösungsmittels, vorteilhaft zwischen 50°C und Rückflusstemperatur des Lösungsmittels.
Nach beendeter Reaktion kann das Imidazopyridin aufübliche Weise aus dem Reaktionsgemisch abgetrennt werden.

### Beispiel 1

### a] Verfahren zur Herstellung von Propionimidsäuremethylester

Zu einer Lösung von 220 g (4 mol) Propionitril und 128,4 g (4 mol) Methanol in 400 ml Diethylether wurde bei 0°C 220 g HCl (6 mol) eingeleitet. Nach beendeter Zugabe wurde das Reaktionsgemisch bei 0°C weitere 16 Stunden gerührt. Der kristalline Feststoff wurde filtriert und mit Ether 2mal gewaschen. Nach Trocknung am Hochvakuum verblieben 460 g (93%) des Titelproduktes als Hydrochlorid.
¹H-NMR (DMSO, 300 MHz) δ 1,15 (t, 3H)
2,71 (q, 2H)
4,1 (s, 3H)
11,2 (breites s, 1H)
12,2 (breites s, 1H)

Zur Freisetzung des Titelproduktes wurde die Reaktionsmischung auf 2N K₂CO₃-Lösung gegossen. Nach Phasentrennung, Abdestillation des Ethers blieb das freie Imidat als farblose Flüssigkeit zurück.
Sdp.: 88 - 92°C
¹H-NMR (CD₃OD, 400 MHz) δ 1,1 (t, 3H)
2,3 (q, 2H)
3,65 (s, 3H)
4,85 (s, 1H)

### b] Verfahren zur Herstellung von 2-Ethyl-5,7-dimethyl-3H-imidazo[4,5 - b]pyridin

Eine Lösung von 4,36 g (50 mmol) des Produktes aus 1a und 50 g (500 mmol; 10eq) Acetylaceton in 50 ml Toluol wurde auf 70°C erhitzt und mit einer filtrierten Lösung von 4,72 g (50 mmol) Aminoacetonitrilhydrochlorid und 2 g (50 mmol) NaOH in 30 ml Methanol versetzt. Man liess 4 Stunden bei 70°C rühren und erhitzte anschliessend langsam bis auf 110°C, wobei das Methanol und Wasser abdestilliert wurde. Nach einer weiteren Stunde bei Rückflusstemperatur, liess man auf Raumtemperatur abkühlen und engte am Rotationsverdampfer das Lösungsmittel ein. Der feste Rückstand wurde in wenig heissem Essigester gelöst, heiss filtriert und anschliessend wieder auf Raumtemperatur abgekühlt. Der auskristallisierte Feststoffwurde abfiltriert, mit wenig kaltem Essigester gewaschen und am Vakuum getrocknet. Man erhielt 4,61 g (52%) leicht gelbes Titelprodukt.
Schmelzpunkt 148,8 - 150,4°C
¹H-NMR (CD₃OD, 400 MHz) δ 1,4 (t, 3H)
2,55 (s, 6H)
2,9 (q, 2H)
6,9 (s, 1H)

### Beispiel 2

### Verfahren zur Herstellung von 2-Ethyl-5,6,7-trimethyl-3H-imidazo[4,5 - b]pyridin

Eine Lösung von 13,3 g (0,15 mol) Propanimidsäuremethylester (Produkt aus Beispiel 1a) und 33,57 g (0,285 mol) 3-Methyl-2,4-pentandion in 150 ml Toluol wurden auf 65°C erhitzt und mit einer Suspension von 13,9 g (0,15 mol) Aminoacetonitril-hydrochlorid und 6 g (0,15 mol) NaOH in 80 ml Methanol versetzt. Man liess 2 Stunden bei 65°C-70°C rühren und destillierte anschliessend das Methanol ab. Es wurde auf Raumtemperatur gekühlt, mit 50 ml Wasser versetzt und mit conc. HCl der pH auf 1,3 gestellt. Die Phasen wurden getrennt und die wässrige Phase mit NaOH-Lösung wieder auf pH 8,4 gebracht. Die Wasserphase wurde mit Ethylacetat mehrere Male extrahiert, die gemeinsame organische Phase über MgSO₄ getrocknet und am Rotationsverdampfer eingeengt. Das Rohprodukt wurde durch Umkristallisation aus Aceton gereinigt. Man erhielt 3,75 g (13%) reines Produkt.
Schmelzpunkt 183,5-184,4°C.
¹H-NMR (CDCl₃, 300 MHz): δ 1,45 (t, 3H)
2,3 (s, 3H)
2,63 (s, 3H)
2,68 (s, 3H)
3,05 (q, 2H)
12,7-13,1 (br, 1H)

### Beispiel 3

### Verfahren zur Herstellung von 2-Cyclopropyl-5,7-dimethyl-3H-imidazo[4,5 - b]pyridin

Zu einer Lösung von 30,13 g (0,2 mol) Cyclopropylimidsäuremethylester-hydrochlorid in 20 ml Methanol wurde bei 0°C langsam 36,0 g (0,2 mol) Natriummethanolat (30%ige Lösung in Methanol) zugetropft. Anschliessend wurde bei der gleichen Temperatur mit 100,1 g (1 mol) Acetylaceton versetzt und dann auf 50°C erhitzt. Zur erhaltenen Suspension wurde eine Suspension von 18,5 g (0,2 mol) Aminoacetonitril-hydrochlorid und 8 g (0,2 mol) NaOH in 50 ml Methanol zugegeben. Man liess 16 Stunden bei 65°C-70°C rühren und destillierte anschliessend das Methanol ab. Die Temperatur stieg allmählich bis auf 110°C an. Man liess noch 3 Stunden bei dieser Temperatur und kühlte anschliessend auf Raumtemperatur ab. Es wurden 150 ml Wasser zugegeben, mit conc. HCl der pH auf 1,3 gestellt und anschliessend mit NaOH wieder neutralisiert. Das Produkt wurde mehrere Male mit Essigester extrahiert, die gemeinsame organische Phase über MgSO₄ getrocknet und am Rotationsverdampfer eingeengt. Das Rohprodukt wurde in 200 ml Ether aufgenommen, gerührt und anschliessend wieder filtriert. Der zurückgebliebene Feststoffwurde in 200 ml Wasser aufgeschlämmt und nach vier Stunden erneut filtriert. Man erhielt 17,75 g Produkt. Dieses wurde aus Toluol umkristallisiert Man erhielt 15,3 g (41%) reines Produkt als gelbliche Kristalle.
Schmelzpunkt 171,7-172,6°C.
¹H-NMR (CD₃OD, 300 MHz): δ 1,1-1,3 (m, 4H)
2,1-2,25 (m, 1H)
2,5 (s, 3H)
2,55 (s, 3H)
6,9 (s, 1H)

### Beispiel 4

### Verfahren zur Herstellung von 2-Ethyl-5-methyl-7-phenyl-3H-imidazo[4,5 - b]pyridin und 2-Ethyl-7-methyl-5-phenyl-3H-imidazo[4,5 - b]pyridin

Eine Lösung von 3,6 g (40 mmol) Propanimidsäuremethylester (Produkt aus Beispiel 1a) und 14,6 g (90 mmol) Benzoylaceton in 50 ml Xylol wurden auf 70°C erhitzt und mit einer Suspension von 3,7 g (40 mmol) Aminoacetonitril-hydrochlorid und 1,6 g (40 mmol) NaOH in 40 ml Methanol versetzt. Man liess 4 Stunden bei 65°C-70°C rühren und destillierte anschliessend das Methanol ab. Die Temperatur stieg allmählich auf 130°C an. Es wurde auf Raumtemperatur gekühlt, mit 150 ml Wasser versetzt und mit conc. HCl der pH auf 2,2 gestellt. Es wurden 100 ml Ethylacetat zugegeben, die Phasen getrennt und die wässrige Phase mit NaOH-Lösung wieder auf pH 7,2 gebracht. Die Wasserphase wurde mit Ethylacetat mehrere Male extrahiert, die gemeinsame organische Phase über MgSO₄ getrocknet und am Rotationsverdampfer eingeengt. Man erhielt 5,05 g Rohprodukt, welches die isomeren Titelverbindungen im Verhältnis von ca. 4:1 enthielt (gemäss ¹H-NMR-Spektrum). Durch Säulenchromatographie (Ethylacetat / Hexan 5:1) wurde das 5-Methyl-7-phenyl-Derivat als Hauptisomer isoliert. Man erhielt 0,73 g als Hauptfraktion, sowie 1,26 g einer Mischfraktion.
Schmelzpunkt 187,4-190,4°C.
¹H-NMR (DMSO, 400 MHz) (vom 5-Methyl-7-phenyl-Isomer): δ 1,4 (t, 3H)
2,6 (s, 3H)
2,9 (q, 2H)
7,34-7,52 (m, 3H)
7,6 (s, 1H)
8,06-8,12 (m, 2H)

## Patentansprüche

1. Verfahren zur Herstellung von Imidazopyridinderivaten der allgemeinen Formel 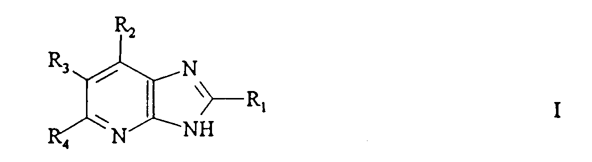 worin
R₁ eine Alkyl-, Cycloalkyl, Aryl- oder Aralkylgruppe bedeutet oder für einen heterocyclischen Rest steht
R₂ und R₄ gleich oder verschieden sind und Wasserstoff, eine Hydroxy-, eine Cyan-, Alkyl-, Cycloalkyl-, Aryl- oder Alalkylgruppe bedeuten oder für eine Alkanoyl - oder eine Alkoxycarbonylgruppe stehen und
R₃ Wasserstoff, eine Alkyl-, Aryl- oder Aralkylgruppe oder ein Halogenatom bedeutet, dadurch gekennzeichnet, dass ein Alkoxyimidat der allgemeinen Formel 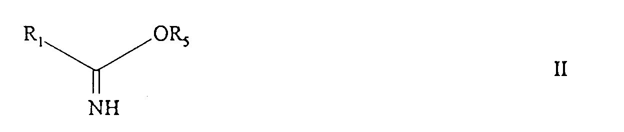
worin R₁ die genannte Bedeutung hat und R₅ Alkyl, Aryl oder Aralkyl bedeutet,mit Aminoacetonitril und mit einer 1,3-Dicarbonylverbindung der allgemeinen Formel 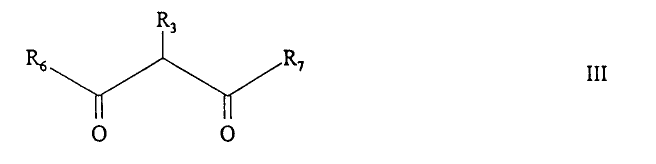 worin R₃ die genannte Bedeutung hat und R₆ und R₇ gleich oder verschieden sind und Wasserstoff, Alkyl, Aryl, Aralkyl, Alkoxy oder Alkoxycarbonyl bedeuten, zum Endprodukt der allgemeinen Formel I ringschlossen wird.

2. Verfahren nach Patentanspruch 1, dadurch gekennzeichnet, dass das Aminoacetonitril aus einem Salz des Aminoacetonitrils mit Hilfe einer Base freigesetzt wird.

3. Verfahren nach Patentanspruch 1 oder 2, dadurch gekennzeichnet, dass der Ringschluss bei einer Temperatur zwischen Raumtemperatur und Rückflusstemperatur des Reaktionsgemisches und gegebenenfalls in Gegenwart eines zusätzlichen Lösungsmittels erfolgt.

4. Verfahren nach Patentanspruch 1, dadurch gekennzeichnet, dass das Alkoxyimidat der allgemeinen Formel II durch Umsetzung eines Nitrils der allgemeinen Formel
R₁CN IV
worin R₁ die genannte Bedeutung hat,mit einem Alkohol der allgemeinen Formel
R₅OH V
worin R₅ die genannte Bedeutung hat, in Gegenwart von einem Halogenwasserstoff gewonnen wird.

## Claims

1. A process for the preparation of imidazopyridine derivatives of the general formula 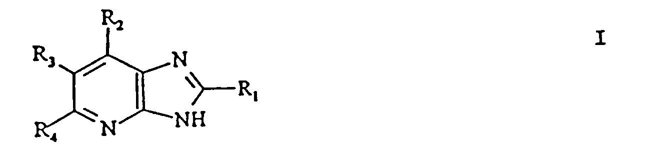 wherein
R₁ is an alkyl, cycloalkyl, aryl or aralkyl group or is a heterocyclic radical,
R₂ and R₄ are identical or different and are hydrogen, a hydroxy, cyano, alkyl, cycloalkyl, aryl or aralkyl group or are an alkanoyl or an alkoxy-carbonyl group, and
R₃ is hydrogen, an alkyl, aryl or aralkyl group or a halogen atom, characterized in that an alkoxy imidate of the general formula 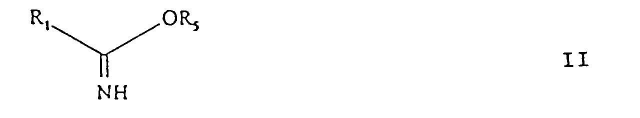
wherein R₁ is as defined above and R₅ is alkyl, aryl or aralkyl, is cyclized with aminoacetonitrile and a 1,3-dicarbonyl compound of the general formula 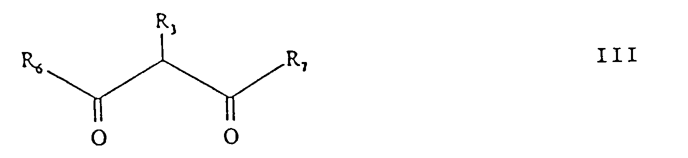 wherein R₃ is as defined above, and R₆ and R₇ are identical or different and are hydrogen, alkyl, aryl, aralkyl, alkoxy or alkoxycarbonyl, to give the final product of the general formula I.

2. The process according to claim 1, characterized in that the aminoacetonitrile is liberated from a salt of aminoacetonitrile by means of a base.

3. The process according to claim 1 or 2, characterized in that the cyclization is carried out at a temperature between room temperature and the reflux temperature of the reaction mixture, and is optionally conducted in the presence of an additional solvent.

4. The process according to claim 1, characterized in that the alkoxy imidate of the general formula II is obtained by reaction of a nitrile of the general formula
R₁CN IV
wherein R₁ is as defined above, with an alcohol of the general formula
R₅OH V
wherein R₅ is as defined above, in the presence of a hydrogen halide.

## Revendications

1. Procédé pour la préparation de dérivés d'imidazopyridines de la formule générale 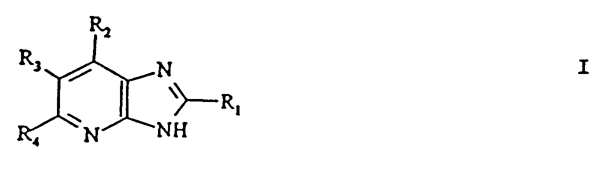 dans laquelle
R₁ signifie un groupe alkyle, cycloalkyle, aryle ou aralkyle ou un radical hétérocyclique
R₂ et R₄ sont identiques ou différents et signifient l'hydrogène, un groupe hydroxy
cyano- alkyle, cycloalkyle, aryle ou aralkyle ou un groupe alcanoyle ou alcoxycarbonyle et
R₃ signifie l'hydrogène, un groupe alkyle, aryle ou aralkyle ou un atome d'halogène, caractérisé en ce que l'on cyclise un alcoxyimidate de la formule générale 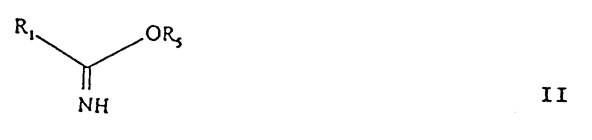
dans laquelle R₁ a la signification ci-dessus et R₅ signifie alkyle, aryle ou aralkyle, avec un aminoacétonitrile et avec un composé 1,3-dicarbonyle de la formule générale 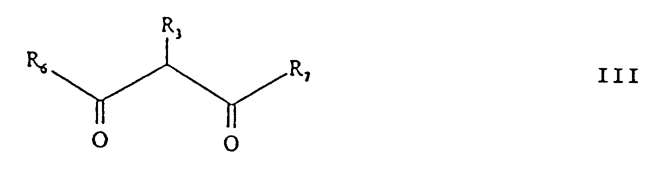 dans laquelle R₃ a la signification citée et R₆ et R₇ sont identiques ou différents et signifient l'hydrogène, alkyle, aryle, aralkyle, alcoxy ou alcoxycarbonyle, pour obtenir le produit final de la formule générale I.

2. Procédé selon la revendication 1, caractérisé en ce que l'aminoacétonitrile est libéré à partir d'un sel d'aminoacétonitrile à l'aide d'une base.

3. Procédé selon la revendication 1 ou 2, caractérisé en ce que la cyclisation s'effectue à une température entre la température ambiante et la température de reflux du mélange réactionnel et éventuellement, en présence d'un solvant supplémentaire.

4. Procédé selon la revendication 1, caractérisé en ce que l'alcoxyimidate de la formule générale II est obtenu par réaction d'un nitrile de la formule générale
R₁CN IV
dans laquelle R₁ a la signification citée, avec un alcool de la formule générale
R₅OH V
dans laquelle R₅ a la signification citée, en présence d'un acide halohydrique
